Europäisches Patentamt

European Patent Office

Office européen des brevets

(11) Veröffentlichungsnummer: **0 006 592 B1**

(12) **EUROPÄISCHE PATENTSCHRIFT**

(45) Veröffentlichungstag der Patentschrift:
12.08.81

(51) Int. Cl.³: **C 13 K 13/00**, C 07 C 29/74

(21) Anmeldenummer: 79102074.6

(22) Anmeldetag: 22.06.79

(54) Verfahren zur Gewinnung von Polyalkoholen, insbesondere Xylit.

(30) Priorität: 22.06.78 DE 2827477

(43) Veröffentlichungstag der Anmeldung:
09.01.80 Patentblatt 80/1

(45) Bekanntmachung des Hinweises auf die Patenterteilung:
12.08.81 Patentblatt 81/32

(84) Benannte Vertragsstaaten:
AT BE CH DE FR GB IT LU NL SE

(56) Entgegenhaltungen:
DE-A-1 165 565
DE-A-2 418 800
**CHEMICAL ABSTRACTS, Vol. 84, 1976
Columbus, Ohio, US
Seite 107, Zusammenfassung Nr. 123699b**

(73) Patentinhaber: **Benckiser-Knapsack GmbH, Dr.
Albert-Reimann-Strasse 2, D-6802 Ladenburg (DE)**

(72) Erfinder: **Riehm, Theodor, Dr. Dipl.-Chem.,
Bothestrasse 30, D-6900 Heidelberg 1 (DE)**
Erfinder: **Auel, Theodor, Dr. Dipl.-Chem.,
Neckarstrasse 58, D-6804 Ilvesheim (DE)**
Erfinder: **Spatz, Wilhelm, Chem.-Ing. grad.,
Talstrasse 125, D-6101 Ober-Kainsbach/Reichelsheim
(DE)**

(74) Vertreter: **Zellentin, Rüdiger, Dr. et al,
Zweibrückenstrasse 15, D-8000 München 2 (DE)**

## Verfahren zur Gewinnung von Polyalkoholen, insbesondere Xylit

Gegenstand der vorliegenden Erfindung ist ein Verfahren zur Gewinnung von Polyalkoholen, insbesondere Xylit, aus Laubhölzern oder einjährigen Pflanzen durch Aufschluß mit verdünnter Mineralsäure bei höheren Temperaturen, Entfärbung und Entionisierung mittels Ionenaustauscher sowie Hydrierung.

Es sind viele Verfahren bekannt, um insbesondere Xylose und Xylit aus Laubhölzern oder einjährigen Pflanzen zu gewinnen. Die meisten Verfahren, bevorzugt solche, die technisch auch durchgeführt werden, laufen auf die folgende Serie von Prozeßstufen hinaus (vgl. »Die Hefen«, Bd. II, S. 82 – 113 (1962), Festkrift Tillägnad Gunnar Sundblad, 10. Oktober 1958, Ivar Haeggströms Boktryckeri AB, S. 75 ff., DE-AS 1 643 940, 2 530 386, 2 350 668, FR-PS 1 477 305):

1. Saure Hydrolyse des pflanzlichen Materials mit verdünnten Mineralsäuren, insbesondere Schwefelsäure oder Salzsäure, bei 100 bis ca. 135° C.
2. Neutralisation mit Kalk und Gipsfiltration (nur bei schwefelsaurer Hydrolyse).
3. Entfernung von Salzen, organischen Säuren, insbesondere Essigsäure und Farbstoffen mittels Ionenaustauschern, Kohle und Entfärbungsharzen.
4. Eindampfung und Kristallisation der Xylose.
5. Hydrierung der Xylose mit Raney-Nickel bei 30 bis 50 atü nach vorheriger pH-Einstellung. Auch die Xylose-Mutterlauge wird in der Regel zu Polyalkoholen hydriert.
6. Nochmalige Entionisierung und Eindampfung der Xylit-Lösung bzw. der Polyalkohol-Lösung.
7. Kristallisation und Trocknung des Xylits.

Varianten der beschriebenen Prozeß-Stufen bestehen außerdem noch in der alkalischen oder sauren oder alkoholischen Vorwaschung vor der eigentlichen Hydrolyse, wie z. B. aus den DE-OS 2 358 407 und 2 522 761 ersichtlich ist. In der DE-OS 2 418 800 wird die chromatographische Fraktionierung der Hydrolyse-Lösung bzw. deren Hydrierungsprodukte beschrieben. Diese Fraktionierung der einzelnen Zucker bzw. Zuckeralkohole bringt zwar reinere Zucker- bzw. Polyalkohollösungen, jedoch keine vollständige Trennung der Xylose von den Zuckern bzw. des Xylit von den Polyalkoholen. Die Isolierung der Xylose aus der xylosereichen — bzw. des Xylits aus der xylitreichen — Fraktion muß durch eine anschließende Kristallisation erfolgen. Für das erfindungsgemäße Verfahren sind diese Prozeß-Stufen nicht erforderlich.

In jedem Falle ist die technische Herstellung von Xylit nur in einer Serie von vielen Prozeß-Schritten und damit hohen Herstellkosten möglich, die die weitere Verbreitung dieses Zuckeraustauschstoffes bisher verhindert haben, obwohl sein angenehmer Geschmack, seine Süßkraft wie Saccharose, seine Verträglichkeit für Diabetiker, vor allem seine nachgewiesenen Antikaries-Eigenschaften und seine gesundheitliche Unbedenklichkeit den Xylit zu einem volkswirtschaftlich sehr interessanten Stoff machen.

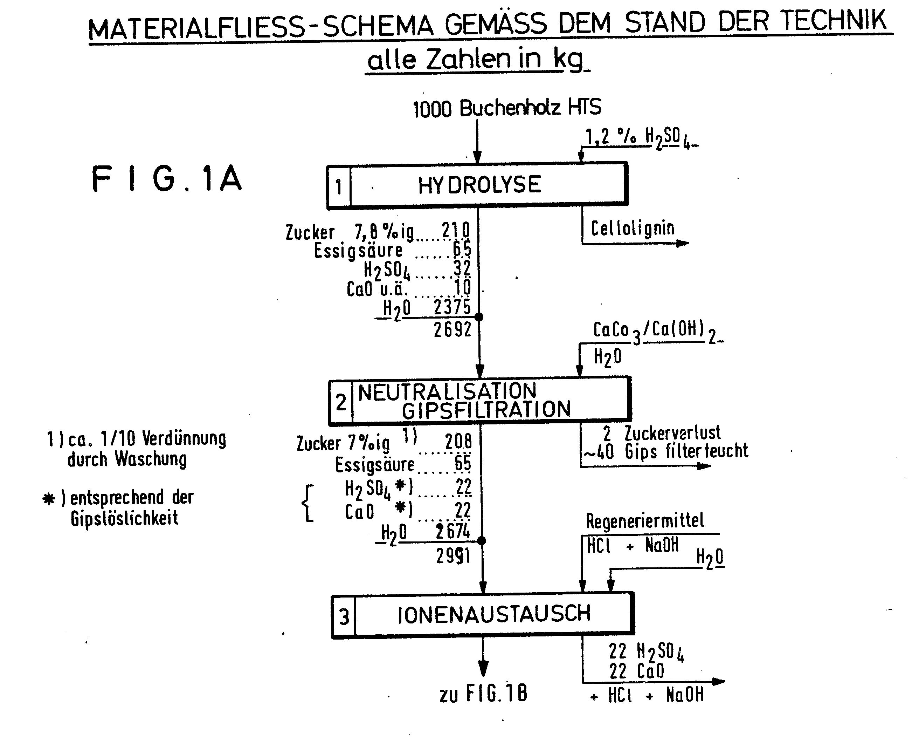

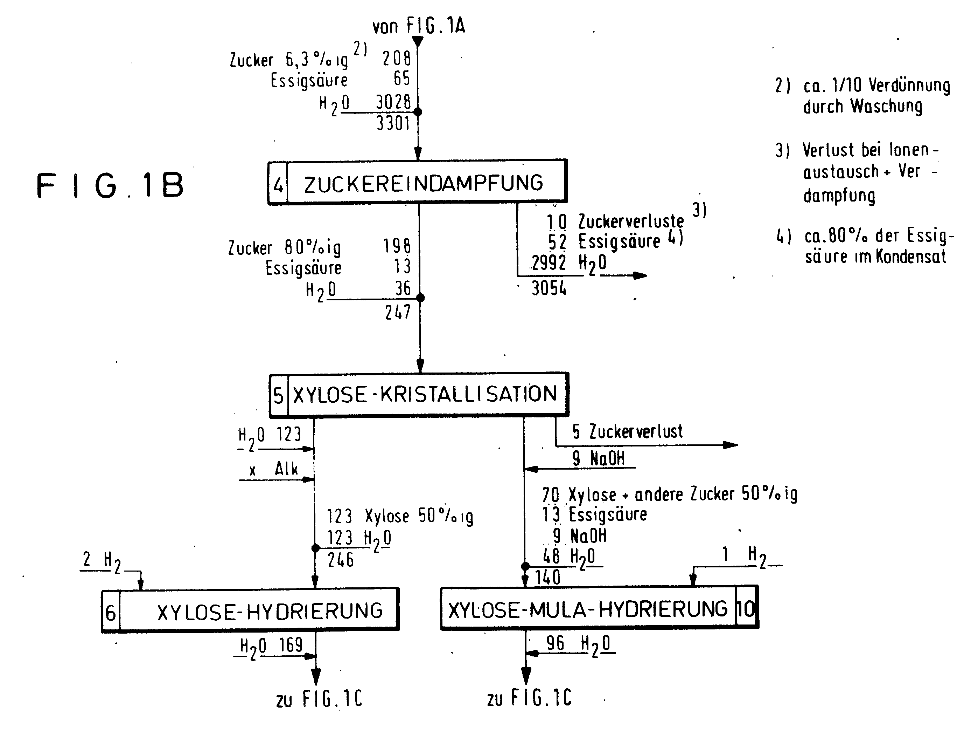

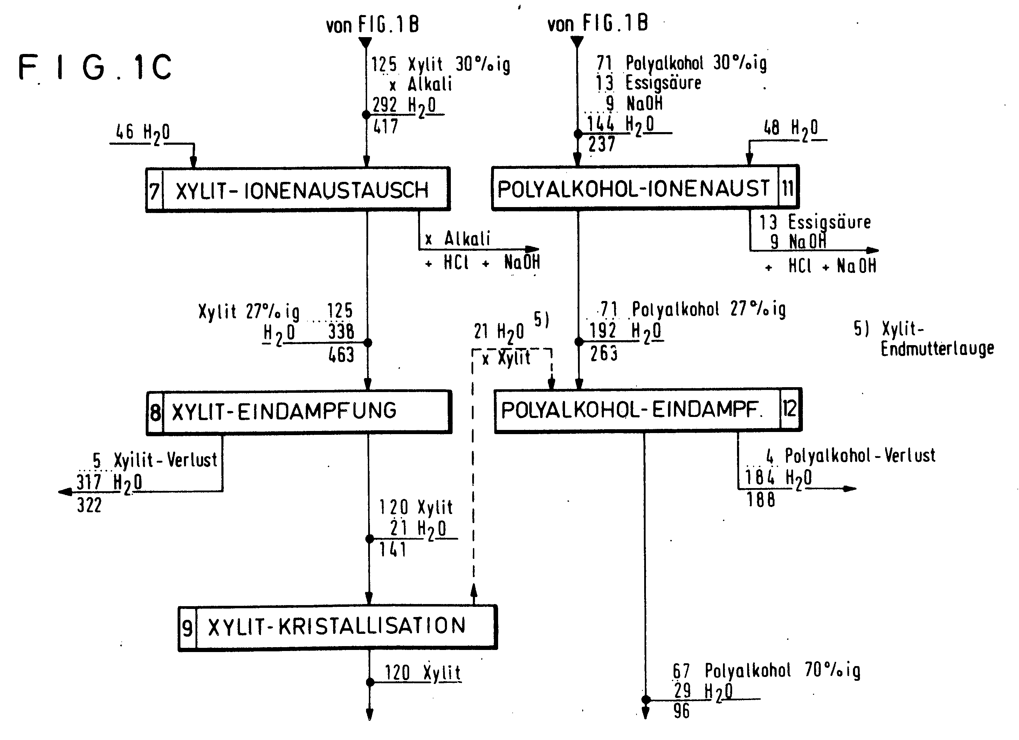

Die vielen oben erwähnten Prozeß-Stufen sind in Form eines typischen, praxisgerechten Beispiels als Materialfließschema in der Abbildung 1 dargestellt.

Die bekannten Prozeß-Stufen oder Anlageteile sind hier in ihrer Folge von oben nach unten in Kastenform aufgeführt. Die verbindenden Linien sind Mengenströme, an denen die angeschriebenen Zahlen jeweils kg, bezogen oder umgerechnet auf 1000 kg Holz-Trockensubstanz, bedeuten. Die Hydrolyse erfolgt mit einer bereits zuckerhaltigen, etwa 1,2prozentigen Schwefelsäure bei 120 bis 130° C. Durch Rückführung von Waschwässern werden Hydrolysate mit ca. 7,8% Zucker mit den in der Abbildung 1 angegebenen Mengen an Essigsäure, Schwefelsäure und Kationen (berechnet als CaO) erhalten.

Nach durchgeführten Untersuchungen bleiben nach der Neutralisation mit Kalk und Filtration des dabei anfallenden Gipses wegen der erhöhten Gipslöslichkeit in Gegenwart von Zucker die in Abbildung 1 angegebenen 22 kg Schwefelsäure und 22 kg CaO-Mengen in Lösung, wobei der CaO-Gehalt sich gegenüber der ursprünglichen Hydrolyse-Lösung mehr als verdoppelt hat. Durch das Auswaschen des Gipses und nach Hinzufügen des Waschwassers wird die Zuckerlösung um ungefähr $1/10$ verdünnt. Außerdem tritt ein Zuckerverlust von etwa 1% auf.

In der Abbildung 1 ist der Ionenaustausch nach den Angaben in der DE-PS 1 183 870 berücksichtigt, wobei 65 kg Essigsäure in Lösung bleiben, um den kostspieligen Anionenaustausch der Essigsäure zu vermeiden.

In der Zucker-Eindampf-Stufe wird dann auf ca. 80% Zucker-Gehalt eingedampft, wobei ca. 80% der Essigsäure abdestillieren und — zusammen mit dem Ionenaustausch-Prozeß — nochmals ein ca. 5prozentiger Zucker-Verlust auftritt.

Die nachfolgende Xylose-Kristallisation ist in Abbildung 1 vereinfachend nur als eine Stufe gezeichnet. In Wirklichkeit stellt aber diese Stufe eine ganze Serie von hintereinander durchgeführten Kristallisations-Schritten mit Mutterlaugen-Eindampfung und Mutterlaugen-Rückführung dar, wie sie zur Erreichung der angegebenen Xylose-Ausbeute obligatorisch sind, wodurch nochmals ca. 2,5% Zucker-Verlust auftreten.

Aus der kristallisierten Xylose wird eine ca. 50prozentige wäßrige Lösung hergestellt und zur pH-Einstellung und Pufferung noch Alkali zugesetzt. Anschließend wird in üblicher Weise hydriert.

Nach der Hydrierung wird die Lösung auf ca. 30% verdünnt, damit der Ionenaustausch möglich ist. Dann folgt Eindampfung und Kristallisation, ebenfalls vereinfacht als eine Stufe dargestellt. Sie umfaßt mehrere Kristallisationen und Mutterlaugen-Eindampfungen, wie sie bei der Xylose-Kristallisation zwecks Erzielung einer hohen Xylit-Ausbeute erforderlich sind. Bei dem Ionenaustausch-Prozeß und der Verdampfung in der Xylit-Stufe treten nochmals Verluste von ca. 5% auf.

Zu einer wirtschaftlichen Durchführung des Verfahrens gehört eine Verwertung der Xylose-Mutterlauge, die üblicherweise durch Hydrierung zu Polyalkoholen in hochwertige Endprodukte übergeführt wird. Dies ist auch in Abbildung 1 dargestellt. In der Xylose-Mutterlauge reichert sich die nicht abdestillierte Essigsäure (13 kg) an. Sie wird aus dem Polyalkohol durch Ionenaustausch entfernt.

Die Xylit-End-Mutterlauge wird dem Polyalkohol zugeführt, wie aus Abbildung 1, Anmerkung 5, ersichtlich ist.

Faßt man aus der oben beschriebenen Folge der üblichen Prozeß-Stufen diejenigen Faktoren zusammen, die die Kosten der Herstellung des Xylits und anderer Polyalkohole in erster Linie bestimmen, so sind dies:

1. Die Menge der zu bindenden Kationen und zwar als CaO und NaOH, sowie die Menge der zu bindenden Anionen, als $H_2SO_4$ und Essigsäure.
   Diese Mengen bestimmen die Anlage-Größe der gesamten Austauscher-Anlagen und damit deren Investitions-, Betriebs- und Regenerierkosten. Sie stellen einen sehr bedeutenden Kostenfaktor der gesamten Xylit-Herstellung dar.
2. Die Summe der Zucker-Verluste in den verschiedenen Verfahrens-Stufen.
3. Die Summe des zu verdampfenden Wassers in allen Prozeß-Stufen.
   Diese Menge bestimmt die Investitionskosten aller Eindampf-Anlagen und deren Energie-Verbrauch als weiterer wichtiger Kostenfaktor der Xylit-Herstellung.

Die nachfolgende Tabelle 1 faßt diese Fakten zahlenmäßig, auf 1000 kg Holz-Trockensubstanz bezogen, zusammen.

Tabelle 1

|  | Kationen-austausch kg CaO-Äquival. (CaO + NaOH) | Anionen-austausch kg $H_2SO_4$- Äquival. | Zucker-verluste kg | Wasserver-dampfung kg $H_2O$ |
|---|---|---|---|---|
| Gipsfiltration |  |  | 2 |  |
| Ionenaustausch- und Eindampfung der Zucker-Lösung | 22 | 22 | 10 | 2992 |
| Xylose-Kristallisation |  |  | 5 |  |
| Xylit-Ionenaustausch und Eindampfung | 1 | 1 | 5 | 317 |
| Polyalkohol-Ionenaustausch und Eindampfung | 6 | 12 | 4 | 184 |
| Zusammen: | 29 | 35 | 26 | 3493 |

Die vorliegende Erfindung beruht auf den nachstehend aufgeführten Merkmalen, die zusammen das erfindungsgemäße Verfahren ausmachen.

Mit dem erfindungsgemäßen Verfahren ist es in überraschender Weise gelungen, verschiedene Prozeß-Stufen zu vereinfachen oder ganz einzusparen, wenn man die Entfärbung und den Ionenaustausch der Zuckerlösung und der Polyalkohol-Lösung in der gleichen Austauscher-Anlage durchführt.

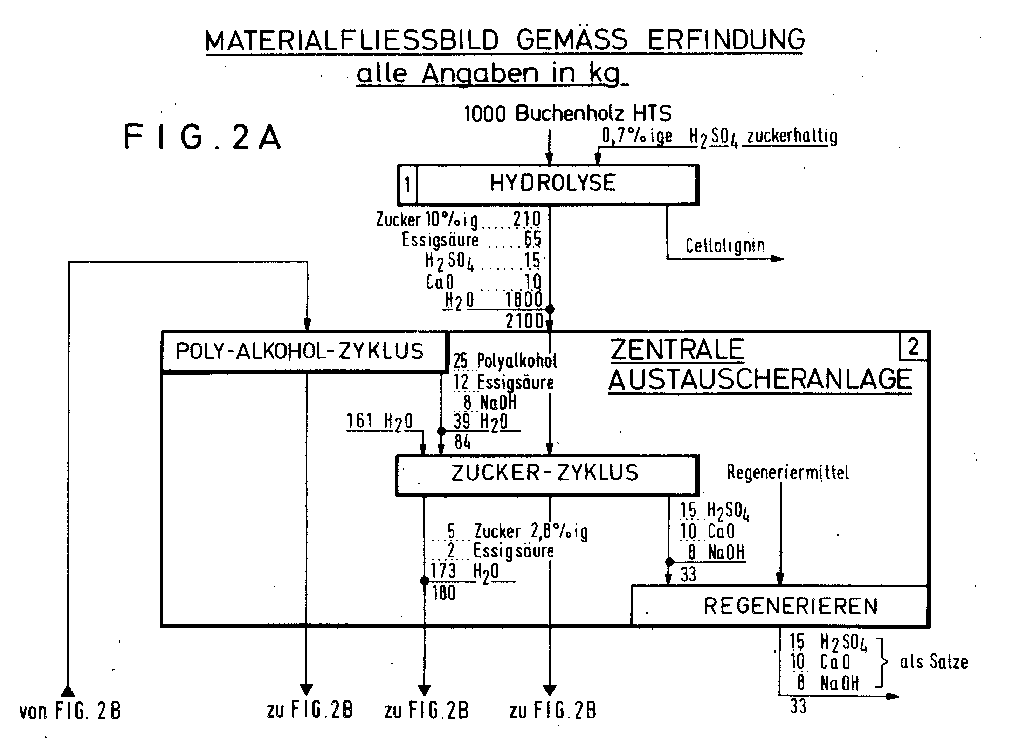

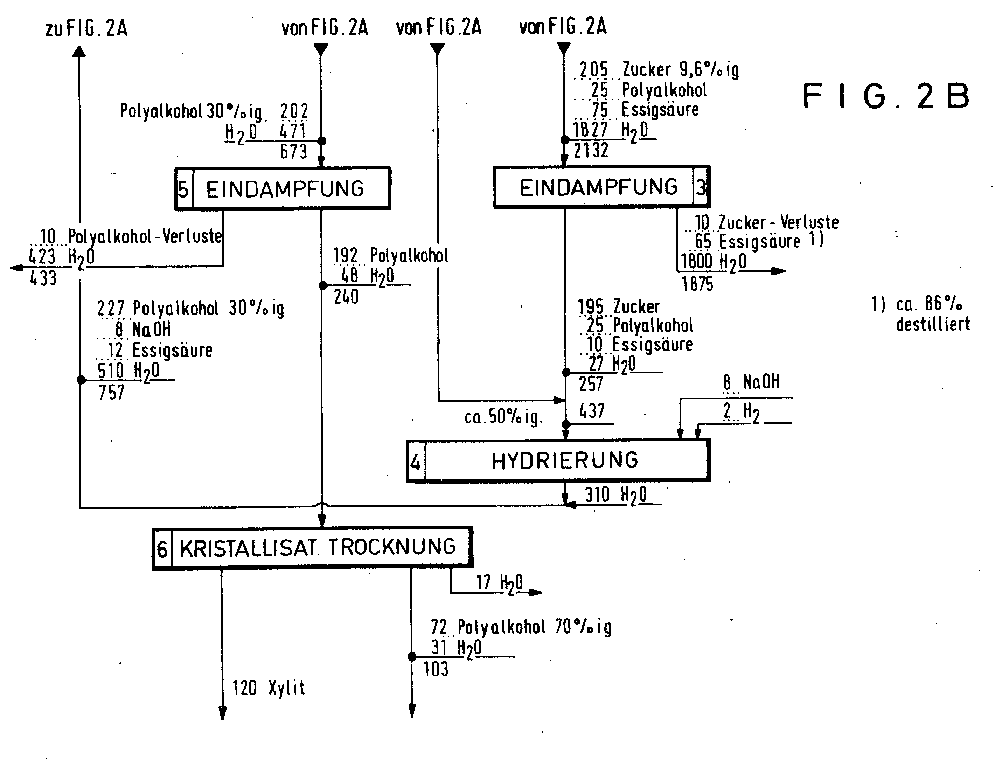

Die einzelnen Verfahrensschritte sind in dem Materialfließschema in Abbildung 2 dargestellt, welches ein gut durchführbares Verfahren darstellt. Alle hier angegebenen Mengen sind entsprechend Abbildung 1 auf eine Eingangs-Holzmenge von 1000 kg Trockensubstanz bezogen, damit ein unmittelbarer Vergleich mit der Abbildung 1 erfolgen kann.

Zur praktischen Durchführung des Verfahrens wird die zum Aufschluß verwendete Schwefelsäure in

3

einer solchen Konzentration eingesetzt, daß eine Neutralisation mittels Calciumcarbonat oder Calciumhydroxyd nicht erforderlich ist. Zur Erreichung dieses Zieles arbeitet man mit einer Schwefelsäure in einer Konzentration von 0,4 bis 0,8%, vorzugsweise 0,7%, bei Temperaturen von 140 bis 160°C, vorzugsweise 150°C, und erhitzt ca. 2 Stunden. Man spart somit nicht nur die Prozeß-Kosten der Gipsfällung und -filtration, sondern man vermeidet auch die höheren Kationenaustauscher-Prozeßkosten, die durch das zugesetzte Neutralisationsmittel üblicherweise verursacht werden. Durch Hintereinanderschalten von mindestens 2 oder 3 Hydrolysetürmen erreicht man ohne größere Zucker-Verluste Zuckerkonzentrationen von etwa 10%, wie aus Abbildung 2 ersichtlich ist.

Die ungewöhnliche Doppelfunktion der Austauscher-Anlage ist mit ganz erheblichen Konsequenzen für Anlagegröße und Herstellungskosten des Xylit-Verfahrens verbunden. Man erreicht so eine erhebliche Einsparung an Investitions-, Regenerier- und Eindampfkosten.

Erfindungsgemäß wird die frisch regenerierte Austauscher-Anlage zunächst mit der nur schwach anionen- und kationenhaltigen und schwach gefärbten Polyalkohol-Lösung beaufschlagt. Durch die zunächst weit überdimensionierte Anlage werden die Polyalkohole vollständig von Kationen und Anionen sowie organischen Säuren und Farbstoffen befreit. Man erhält eine sehr reine Polyalkohol-Lösung, so daß besonders hohe Kristallisations-Ausbeuten, bedingt durch den Reinheitsgrad der Lösung, erzielt werden.

Besonders hervorzuheben ist, daß erfindungsgemäß auch ohne die aufwendige Xylose-Kristallisations-Stufe die gleiche Xylit-Ausbeute von etwa 120 kg erreicht wird.

Aus der Abbildung 2 ist weiterhin ersichtlich, daß die 12 kg Rest-Essigsäure der hydrierten Lösung vom Austauscher gebunden werden und in den Zucker-Zyklus übergehen.

Nach dem Polyalkohol-Zyklus wird die bisher nur schwach belegte Ionenaustausch- und Entfärbungs-Anlage nunmehr mit der nach dem Aufschluß erhaltenen Zuckerlösung mit ihren sehr viel höheren Kationen- und Anionen-Konzentrationen im Zucker-Zyklus bis zur Kapazitätserschöpfung gefahren. Der Gehalt an Kationen beträgt etwa 10 kg als CaO und an Anionen etwa 15 kg als $H_2SO_4$. Somit wird im Zucker-Zyklus eine vollständige Kapazitätsausnutzung der Austauscher-Anlage erreicht, wobei geringe, in der Abbildung 2 nicht berücksichtigte Ionendurchbrüche nicht nachteilig sind, weil keine Xylose-Kristallisations-Stufe nachgeschaltet ist. Eventuell durchgebrochene Spuren von Ionen werden bei der Endreinigung im Polyalkohol-Zyklus vor der Xylit-Kristallisation entfernt.

In dem Zucker-Zyklus wird die während des Polyalkohol-Zyklusses absorbierte Essigsäure (12 kg) und der gebundene Anteil an Essigsäure aus der Austauscher-Anlage durch die stärkere Schwefelsäure (15 kg) vollständig eluiert. Dadurch wird ermöglicht, daß die gesamte organische Säure (65 kg) nicht mehr durch den teuren Ionenaustausch, sondern in der nachfolgenden Eindampf-Stufe durch Destillation aus der Zuckerlösung entfernt wird. Eine geringe Menge organischer Säure, nach Abbildung 2 (10 kg), bleibt zwar in der Zuckerlösung zurück, wird aber rezirkuliert und letztlich dann doch durch Destillation abgeschieden. Die Abtrennung der Essigsäure durch Destillation ist ungleich viel billiger als durch Anionen-Austausch, zumal die Destillation zur Erreichung einer höheren Zucker-Konzentration ohnehin obligatorisch ist. Die Essigsäure kann dann durch Rektifikation oder Flüssig-Flüssig-Extraktion aus dem Kondensat gewonnen werden.

Nach dem Zucker-Zyklus wird vor der Regenerierung die Austauscher-Anlage wie üblich mit 161 kg Wasser ausgewaschen, damit kein Zucker mit den Regenerierwässern verlorengeht. Zum Unterschied von der üblichen Fahrweise wird die Austauscher-Anlage trotz ihrer Doppelfunktion nur noch bei dem Zucker-Zyklus mit Wasser gewaschen. Beim Übergang von dem Polyalkohol-Zyklus auf den Zucker-Zyklus wird der spezifisch schwerere Polyalkohol von oben nach unten durch die leichtere Zuckerlösung aus dem Harzbett verdrängt. Bei dem recht scharfen Übergang von Polyalkohol auf Zucker im unteren Ausfluß der Austauscher-Anlage kann sehr schnell von »Eindampfung Polyalkohol« (linker Produktionsstrom der Abbildung 2) auf Zucker umgeschaltet werden, so daß Spuren von Polyalkohol in den Zuckerstrom, nicht aber Zucker in den Polyalkoholstrom kommen können. Dieser Polyalkohol-Gehalt der Zuckerlösung ist ohne Bedeutung, da er keine erhöhte Austauscher-Belastung verursacht. Hierdurch wird durch Vermeidung der zweimaligen Auswaschung die insgesamt zu verdampfende Wassermenge verringert. Nur bei der Hydrierung entsteht eine zusätzliche, aber im Verfahren bedeutungslose Reaktor-Volumen-Belastung durch die kleine, im Kreislauf geführte Polyalkohol-Menge, die durch eine nur wenig erhöhte Gesamt-Konzentration abgefangen werden kann.

Zur Erreichung einer hohen Essigsäure-Destillationsrate wird die Eindampfung wesentlich weiter getrieben, als dies für die Hydrierung zweckmäßig ist, die üblicherweise in 50%igen Lösungen durchgeführt wird. Eine technologische Grenze wird durch die Viskosität der Zuckerlösung gesetzt. Hier wird gemäß Abbildung 2 bei 85prozentiger Zucker + Polyalkohol-Lösung eine Destillationsrate von ca. 86% der Essigsäure erreicht.

Um die 85prozentige Lösung für die Hydrierung auf 50% zu verdünnen, wird anstelle von frischem Wasser die Waschlösung, die ohnehin einzudampfen wäre, aus dem Zucker-Zyklus der Austauscher benutzt. Gemäß Abbildung 2 werden 180 kg einer Lösung, die etwa 5 kg Zucker enthält, aus der Austauscher-Anlage direkt zur Verdünnung der 85prozentigen Zuckerlösung verwendet. Dadurch muß nicht mehr Wasser verdampft werden, als wenn die Gesamt-Lösung auf 50% eingedampft worden wäre.

4

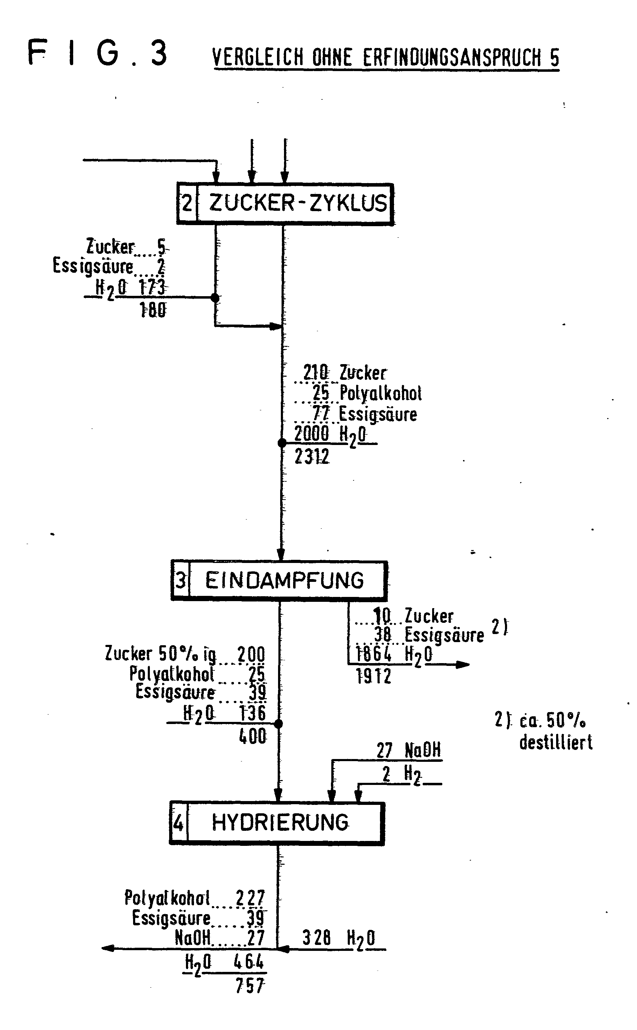

In der Abbildung 3 ist der ungünstigere Fall zum Vergleich dargestellt. Hierbei würden also 39 kg Essigsäure statt 10 kg rezirkuliert, ferner würden zur Neutralisation der Essigsäure 27 kg statt 8 kg NaOH benötigt, die später sogar wieder durch Kationen-Austausch zu eliminieren wären.

Nachstehend sind in Tabelle 2 die kostenbeeinflussenden Faktoren des erfindungsgemäßen Verfahrens im Vergleich zu dem üblichen Verfahren gemäß Tabelle 1 zusammengestellt:

Tabelle 2

| | Kationen-austausch kg CaO-Äquival. (CaO + NaOH) | Anionen-austausch kg $H_2SO_4$-Äquival. | Zucker-verluste kg | Wasserver-dampfung kg $H_2O$ |
|---|---|---|---|---|
| Zentraler Ionenaustausch | 15 | 15 | | – |
| Zuckerverdampfung | | | 10 | 1800 |
| Polyalkoholverdampfung | | | 10 | 423 |
| Polyalkohol-Nachverdampfung | | | | 17 |
| Zusammen: | 15 | 15 | 20 | 2240 |
| vgl. Tab. 1 | 29 | 35 | 26 | 3493 |

Nach dem konventionellen Verfahren werden 12 Teilanlagen benötigt, im erfindungsgemäßen Verfahren jedoch nur 6.

Die Gegenüberstellung zeigt, daß im erfindungsgemäßen Verfahren die Anzahl der Teilanlagen auf die Hälfte reduziert ist. Zusätzlich wird eine dem CaO bzw. $H_2SO_4$ entsprechende Volumenverringerung der Austauscher-Anlage um 48 bzw. 57% erreicht. Außerdem reicht entsprechend der ausgewiesenen $H_2O$-Verdampfung ein Verdampfer, dessen Auslegung und Energiebedarf um 36% niedriger ist. Des weiteren reduziert sich der Zucker-Verarbeitungsverlust durch die verminderte Prozeß-Stufenzahl um ca. 23%.

**Patentansprüche**

1. Verfahren zur Gewinnung von Polyalkoholen, insbesondere Xylit, aus Laubhölzern oder einjährigen Pflanzen durch Aufschluß mit verdünnter Mineralsäure bei Temperaturen oberhalb 100° C, Entfärbung und Entionisierung mittels Ionenaustauscher und Hydrierung, dadurch gekennzeichnet, daß die Entfärbung und der Ionenaustausch der Zuckerlösung, die neben dem Zucker vor allem noch Salze und Essigsäure enthält, und der Polyalkohollösung in der gleichen Austauscher-Anlage durchgeführt werden.

2. Verfahren nach Anspruch 1, dadurch gekennzeichnet, daß man die regenerierte Anlage erst mit Polyalkohol-Lösung beaufschlagt, wobei die gesamte darin enthaltene, aus der Hydrolyse stammende, Essigsäure gebunden wird und anschließend mit Zuckerlösung beschickt, hierdurch die Essigsäure mittels der in der Zuckerlösung enthaltenen Mineralsäure eluiert.

3. Verfahren nach Anspruch 1 und 2, dadurch gekennzeichnet, daß nach dem Polyalkohol-Zyklus die in der Anlage verbleibende, spezifisch schwerere Polyalkohol-Lösung von oben nach unten durch die spezifisch leichtere Zuckerlösung verdrängt wird und die Anlage erst nach dem Zucker-Zyklus mit Wasser gewaschen und regeneriert wird.

4. Verfahren nach Anspruch 1, dadurch gekennzeichnet, daß man die Zuckerlösung wesentlich über die zur Hydrierung übliche Konzentration von 50% aufkonzentriert, hierdurch die im Kreislauf befindliche Essigsäure praktisch, bezogen auf die durch Hydrolyse entstehende Essigsäure, vollständig abtrennt und die Zuckerlösung mit Waschwasser aus dem Zucker-Zyklus der Austauscher-Anlage auf eine zur Hydrierung geeignete Konzentration verdünnt.

5. Verfahren nach Anspruch 1, dadurch gekennzeichnet, daß man die nach Hydrolyse, Ionenaustausch-Reinigung und Eindampfung erhaltene Zuckerlösung insgesamt hydriert und aus der Gesamt-Polyalkohol-Lösung nach nochmaliger Ionenaustausch-Reinigung und Eindampfung Xylit durch Kristallisation gewinnt.

6. Verfahren nach Anspruch 1, dadurch gekennzeichnet, daß man den Aufschluß mit einer 0,4- bis 0,8prozentigen Schwefelsäure bei 140 bis 160° C innerhalb ca. 2 Stunden ohne nachfolgende Neutralisation mit Ca-Salzen durchführt.

7. Verfahren nach Anspruch 6, dadurch gekennzeichnet, daß man den Aufschluß mit einer 0,7prozentigen Schwefelsäure bei 150° C durchführt.

## 0 006 592

### Claims

1. A method of obtaining polyalcohols, in particular xylitol, from hardwood trees or annual plants by decomposition with diluted mineral acid at temperatures over 100°C, decolorization and de-ionization by means of ion exchangers and hydrogenation, characterized in that the decolorization and the ion exchange of the sugar solution, which besides the sugar predominantly contains salts and acetic acid, and of the polyalcohol solution are performed in the same exchanger unit.

2. A method according to claim 1, characterized in that polyalcohol solution is first admitted to the regenerated exchanger unit, the whole of the acetic acid which is contained therein and which originates from the hydrolysis being bonded, and the exchanger unit is subsequently charged with sugar solution, and in this way the acetic acid is eluted by means of the mineral acid contained in the sugar solution.

3. A method according to claims 1 and 2, characterized in that after the polyalcohol cycle the polyalcohol solution of greater specific weight remaining in the unit is forced downwards from above by the sugar solution of lighter specific weight and the unit is washed with water and regenerated only after the sugar cycle.

4. A method according to claim 1, characterized in that the sugar solution is condentrated to substantially above the concentration of 50% usual for hydrogenation, and in this way the acetic acid present in the cycle is separated practically completely relative to the acetic acid produced by hydrolysis and the sugar solution is diluted with washing water from the sugar cycle of the exchanger unit to a concentration suitable for hydrogenation.

5. A method according to claim 1, characterized in that the sugar solution obtained after hydrolysis, ion-exchange purification and evaporation is hydrogenated as a whole and xylitol is obtained by crystallization from the total polyalcohol solution after repeated ion-exchange purification and evaporation.

6. A method according to claim 1, characterized in that decomposition is carried out with 0.4 to 0.8% sulphuric acid at 140 to 160°C within approximately 2 hours without subsequent neutralization with Ca-salts.

7. A method according to claim 6, characterized in that decomposition is carried out with 0.7% sulphuric acid at 150°C.

### Revendications

1. Procédé pour obtenir des polyols, plus spécialement du xylitol, à partir de feuillus ou de plantes annuelles par attaque avec un acide minéral dilué, à des températures supérieures à 100°C, décoloration et désionisation au moyen d'un échangeur d'ions, et hydrogénation, procédé caractérisé en ce qu'on effectue dans la même installation d'échange la décoloration et l'échange d'ions de la solution de sucre qui, en plus du sucre, contient essentiellement encore des sels et de l'acide acétique, et de la solution de polyols.

2. Procédé selon la revendication 1, caractérisé en ce que l'installation régénérée est alimentée d'abord avec la solution de polyols, la totalité de l'acide acétique, provenant de l'hydrolyse, contenu dans cette solution étant alors fixée, et est ensuite alimentée avec la solution de sucre, ce qui a pour effet d'éluer l'acide acétique au moyen de l'acide minéral contenu dans la solution de sucre.

3. Procédé selon l'une des revendications 1 et 2, caractérisé en ce que, après le cycle de polyols, on fait passer la solution de polyols, plus dense, qui reste dans l'installation, de haut en bas, à travers la solution de sucre moins dense, et on ne lave à l'eau et régénère l'installation qu'après le cycle de sucre.

4. Procédé selon la revendication 1, caractérisé en ce que la concentration de la solution de sucre est augmentée à une valeur bien supérieure à la concentration usuelle pour l'hydrogénation, c'est-à-dire 50%, et ainsi l'acide acétique qui se trouve dans le circuit est séparé presque totalement, par rapport à l'acide acétique qui se forme par hydrolyse, et la solution de sucre est diluée avec l'eau de lavage provenant du cycle de sucre de l'installation d'échangeur à une concentration appropriée pour l'hydrogénation.

5. Procédé selon la revendication 1, caractérisé en ce qu'on hydrogène globalement la solution de sucre obtenue après l'hydrolyse, la purification par échange d'ions et l'évaporation et, de la solution de polyols globale, après qu'elle a été purifiée encore une fois par échange d'ions et qu'elle a été évaporée, on isole le xylitol par cristallisation.

6. Procédé selon la revendication 1, caractérisé en ce qu'on effectue l'attaque avec de l'acide sulfurique d'une concentration comprise entre 0,4 et 0,8%, à une température de 140 à 160°C, en 2 heures environ, sans neutraliser ultérieurement avec des sels de calcium.

7. Procédé selon la revendication 6, caractérisé en ce que l'attaque est effectuée à 150°C au moyen d'un acide sulfurique à 0,7%.

# MATERIALFLIESS-SCHEMA GEMÄSS DEM STAND DER TECHNIK
## alle Zahlen in kg

1000 Buchenholz HTS

1,2 % $H_2SO_4$

F I G . 1A

| 1 | HYDROLYSE |

Cellolignin

Zucker 7,8 %ig .... 210
Essigsäure ...... 65
$H_2SO_4$ ...... 32
CaO u.ä. ...... 10
$H_2O$ 2375
2692

$CaCo_3/Ca(OH)_2$
$H_2O$

| 2 | NEUTRALISATION GIPSFILTRATION |

2 Zuckerverlust
~40 Gips filterfeucht

Zucker 7%ig [1] 208
Essigsäure .... 65
$H_2SO_4$ *) .... 22
CaO *) .... 22
$H_2O$ 2674
2991

Regeneriermittel
HCl + NaOH   $H_2O$

1) ca. 1/10 Verdünnung durch Waschung

*) entsprechend der Gipslöslichkeit

| 3 | IONENAUSTAUSCH |

22 $H_2SO_4$
22 CaO
+ HCl + NaOH

zu FIG. 1B

von FIG.1A

F I G .1B

Zucker 6,3 %ig [2]   208
Essigsäure   65
$H_2O$   3028
3301

2) ca. 1/10 Verdünnung durch Waschung

3) Verlust bei Ionen-austausch + Ver-dampfung

4) ca. 80% der Essig-säure im Kondensat

| 4 | ZUCKEREINDAMPFUNG |

1,0 Zuckerverluste [3]
52 Essigsäure [4]
2992 $H_2O$
3054

Zucker 80%ig   198
Essigsäure   13
$H_2O$   36
247

| 5 | XYLOSE-KRISTALLISATION |

$H_2O$ 123

x   Alk

5 Zuckerverlust

9 NaOH

123 Xylose 50%ig
123 $H_2O$
246

70 Xylose + andere Zucker 50%ig
13 Essigsäure
9 NaOH
48 $H_2O$
140

2 $H_2$

1 $H_2$

| 6 | XYLOSE-HYDRIERUNG |

| XYLOSE-MULA-HYDRIERUNG | 10 |

$H_2O$ 169

96 $H_2O$

zu FIG.1C

zu FIG.1C

0 006 592

FIG.1C

125 Xylit 30%ig
x Alkali
292 $H_2O$
417

71 Polyalkohol 30%ig
13 Essigsäure
9 NaOH
144 $H_2O$
237

46 $H_2O$

48 $H_2O$

| 7 | XYLIT-IONENAUSTAUSCH |

| POLYALKOHOL-IONENAUST | 11 |

x Alkali
+ HCl + NaOH

13 Essigsäure
9 NaOH
+ HCl + NaOH

Xylit 27%ig   125
$H_2O$   338
463

21 $H_2O$  5)
x Xylit

71 Polyalkohol 27%ig
192 $H_2O$
263

5) Xylit-
Endmutterlauge

| 8 | XYLIT-EINDAMPFUNG |

| POLYALKOHOL-EINDAMPF. | 12 |

5 Xyilit-Verlust
317 $H_2O$
322

4 Polyalkohol-Verlust
184 $H_2O$
188

120 Xylit
21 $H_2O$
141

| 9 | XYLIT-KRISTALLISATION |

120 Xylit

67 Polyalkohol 70%ig
29 $H_2O$
96

0 006 592

# MATERIALFLIESSBILD GEMÄSS ERFINDUNG
## alle Angaben in kg

**F I G . 2 A**

1000 Buchenholz HTS

0,7%ige $H_2SO_4$ zuckerhaltig

| 1 | HYDROLYSE |

Zucker 10%ig .... 210
Essigsäure ...... 65
$H_2SO_4$ ...... 15
CaO ...... 10
$H_2O$ ...... 1800
2100

Cellolignin

POLY-ALKOHOL-ZYKLUS

**ZENTRALE AUSTAUSCHERANLAGE** | 2 |

25 Polyalkohol
12 Essigsäure
8 NaOH
161 $H_2O$    39 $H_2O$
84

ZUCKER-ZYKLUS

Regeneriermittel

15 $H_2SO_4$
10 CaO
8 NaOH
33

5 Zucker 2,8%ig
2 Essigsäure
173 $H_2O$
180

REGENERIEREN

15 $H_2SO_4$
10 CaO } als Salze
8 NaOH
33

von FIG. 2B    zu FIG.2B    zu FIG.2B    zu FIG.2B

FIG. 2B

0 006 592

zu FIG.2A   von FIG.2A   von FIG.2A   von FIG.2A

205  Zucker 9,6%ig
 25  Polyalkohol
 75  Essigsäure
Polyalkohol 30%ig  202
$H_2O$  471        1827  $H_2O$
          673        2132

| 5 | EINDAMPFUNG |          | EINDAMPFUNG | 3 |

10  Polyalkohol-Verluste
423  $H_2O$          192  Polyalkohol        10  Zucker-Verluste
433                   48  $H_2O$             65  Essigsäure 1)
                      240                   1800  $H_2O$
                                            1875

227  Polyalkohol 30%ig                      195  Zucker
  8  NaOH                                     25  Polyalkohol
 12  Essigsäure                               10  Essigsäure         1) ca. 86%
510  $H_2O$                                   27  $H_2O$                destilliert
757                                          257              8  NaOH

                      ca.50%ig.  437         2  $H_2$

| 4 | HYDRIERUNG |

310  $H_2O$

| 6 | KRISTALLISAT. TROCKNUNG |

17  $H_2O$

72  Polyalkohol 70%ig
31  $H_2O$
103

120  Xylit

# F I G . 3  VERGLEICH OHNE ERFINDUNGSANSPRUCH 5

| 2 | ZUCKER-ZYKLUS |

Zucker ..... 5
Essigsäure ..... 2
$H_2O$  173
180

210  Zucker
25  Polyalkohol
77  Essigsäure
2000  $H_2O$
2312

| 3 | EINDAMPFUNG |

10  Zucker
38  Essigsäure  2)
1864  $H_2O$
1912

Zucker 50 % ig ..... 200
Polyalkohol ..... 25
Essigsäure ..... 39
$H_2O$  136
400

2) ca. 50 %
destilliert

27  NaOH
2  $H_2$

| 4 | HYDRIERUNG |

Polyalkohol ..... 227
Essigsäure ..... 39
NaOH ..... 27  328  $H_2O$
$H_2O$  464
757